(19) Europäisches Patentamt
European Patent Office
Office européen des brevets

(11) Numéro de publication: **0 145 588**
**B1**

(12) # FASCICULE DE BREVET EUROPEEN

(45) Date de publication du fascicule du brevet: **17.11.88**

(51) Int. Cl.⁴: **C 07 C 69/54**, C 07 C 67/62, C 07 C 67/03

(21) Numéro de dépôt: **84402465.3**

(22) Date de dépôt: **30.11.84**

(54) **Procédé d'obtention d'acrylates ou de méthacrylates d'alcoyles supérieurs comprenant l'emploi d'aminophénols comme inhibiteur de polymérisation.**

(30) Priorité: **02.12.83 ES 527725**

(43) Date de publication de la demande: **19.06.85 Bulletin 85/25**

(45) Mention de la délivrance du brevet: **17.11.88 Bulletin 88/46**

(84) Etats contractants désignés: **BE DE FR GB IT**

(56) Documents cités:
**FR - A - 1 352 686**
**FR - A - 1 544 542**
**FR - A - 1 568 382**
**FR - A - 2 313 346**
**GB - A - 960 005**
**US - A - 3 686 268**
**US - A - 3 959 358**

(73) Titulaire: **REPSOL PETROLEO S.A., José Abascal, No 4, 28003 Madrid (ES)**

(72) Inventeur: **Hernandez-Vaquero Espinosa, Mariano, Sacramento 10, Madrid (ES)**
Inventeur: **Gonzalez Nuevo, Amando, Plaza de las Meninas 3, Madrid (ES)**
Inventeur: **Delgado Oyague, Juan Antonio, Avda. de Burgos 30, Madrid (ES)**

(74) Mandataire: **Durand, Yves Armand Louis et al, CABINET WEINSTEIN 20, Avenue de Friedland, F-75008 Paris (FR)**

EP 0 145 588 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un procédé d'obtention d'acrylates ou de méthacrylates d'alcoyles supérieurs comprenant l'emploi d'aminophénoles ou d'aminométhylphénols comme inhibiteurs de polymérisation.

Plus précisément, l'invention concerne des perfectionnements dans un procécé d'obtention d'acrylates et de méthacrylates d'alcoyles supérieurs de pureté élevée au moyen d'une transestérification d'acrylates ou de méthacrylates de méthyle ou d'éthyle avec des alcools contenant plus de deux atomes de carbone, en présence d'alcoxydes de titane comme catalyseurs et d'un inhibiteur de polymérisation et éventuellement en présence d'agents facilitant l'élimination de la masse réactionnelle des alcools méthyliques ou éthyliques produits, au moyen de la formation de mélanges azéotropes.

Il est connu que pour l'obtention d'esters d'alcools supérieurs des acides acryliques et méthacryliques au moyen d'une transestérification des esters méthyliques ou éthyliques correspondants on peut utiliser aussi bien des catalyseurs acides que des catalyseurs basiques. Les catalyseurs acides, tels que l'acide sulfurique ou l'acide p-toluène sulfonique (Journal of Applied Chemistry, volume 13, 1963, pages 168–71) présentent une activité relativement faible et donnent lieu à des réactions secondaires.

Les catalyseurs basiques, tels que les alcoxydes alcalins, décrits par exemple, dans le brevet suisse 239.750, présentent des activités élevées. Cependant, ces composés catalysent simultanément l'addition des alcools sur la double liaison des esters insaturés. Ce fait est un inconvénient de ces catalyseurs étant donné que si on désire aboutir à des sélectivités adéquates en esters insaturés, il est nécessaire de limiter la conversion de la réaction de transestérification, ce qui suppose un grand inconvénient du point de vue industriel.

Les alcoxydes de magnésium sont également des catalyseurs connus de transestérification (brevet allemand n° 1.568.376, 1974). Il s'agit de catalyseurs très actifs dans la réaction de transestérification d'acrylates et de méthacrylates de méthyle et d'éthyle avec des alcools supérieurs. Egalement, ils peuvent se séparer facilement de la masse réactionnelle, une fois terminée la réaction, au moyen d'une filtration, à cause de leur insolubilité élevée. Cependant, ces composés sont très sensibles à la présence de traces d'eau dans la masse réactionnelle, ce qui les rend inactifs d'un point de vue catalytique. Ceci suppose une complication dans la pratique industrielle et un coût supplémentaire de purification des réactifs habituellement utilisés, qui doivent être totalement anhydres.

Dans le brevet allemand 2.524.930 (1976), on propose l'utilisation de cyanures alcalins comme catalyseurs, en concentrations de 0,05–10% en poids. Bien que ces cyanures et en particulier le cyanure de potassium sont de bons catalyseurs de transestérification et qu'ils peuvent se séparer des produits de la réaction au moyen d'une filtration, ils présentent l'inconvénient qu'ils ne peuvent pas être recyclés dans le procécé de transestérification à cause de la perte d'activité catalytique pendant la réalisation de la réaction. Etant donné le danger de ces produits, leur élimination pose un problème important de contamination.

Cet ultime inconvénient ne se présente pas quand on utilise comme catalyseurs des alcoxydes de titane, tels que ceux décrits dans le brevet US n° 2.822.348 (1958). Cependant, avec la technique actuelle, ces catalyseurs présentent l'inconvénient que leur séparation de la masse réactionnelle exige qu'ils soient préalablement hydrolysés pour les rendre insolubles et pouvoir ainsi les séparer au moyen d'une filtration ou d'une centrifugation.

L'activité de ces catalyseurs augmente de manière très marquée avec la température de la réaction de transestérification. Cependant, avec la technique actuelle cette température est limitée par la tendance des monomères acryliques de se polymériser à des températures élevées, de sorte que cela oblige à utiliser des concentrations élevées d'inhibiteurs de polymérisation. La présence de concentrations d'inhibiteurs relativement élevées dans les acrylates ou méthacrylates d'alcoyle est indésirable, car ils peuvent interférer dans les applications postérieures de ces monomères, étant donné qu'on les utilise habituellement comme matière première de différents polymères. Ainsi, on comprend que pendant la polymérisation, la présence de quantités relativement importantes d'inhibiteurs dans les acrylates ou méthacrylates d'alcoyle peut causer des problèmes.

D'autre part, la séparation de l'excès d'inhibiteur des produits finals de la réaction de transestérification au moyen, par exemple, d'extraction avec un solvant adéquat, est une opération supplémentaire indésirable d'un point de vue industriel.

De ce fait, il est nécessaire en l'état actuel de la technique de disposer d'inhibiteurs de polymérisation plus efficaces qui permettent de réaliser la réaction de transestérification à des températures plus élevées, avec des concentrations d'inhibiteurs qui n'exigent pas leur séparation de la masse réactionnelle par le fait qu'ils n'interfèrent pas dans les étapes postérieures de polymérisation des monomères acryliques ou méthacryliques obtenus.

Le brevet USA-3.686.268 concerne un procédé de transestérification utilisant des phenoxydes de titane comme catalyseurs et stabilisants de polymérisation. L'emploi de ces composés présente le désavantage d'incorporer dans le procédé l'étape supplémentaire de leur préparation; celle-ci peut se faire par exemple à partir des titanates d'alkyle et des phénols correspondants, les aminophénols étant mentionnés de façon générale mais non spécifiée.

La présente invention a donc pour objet de perfectionner un procédé selon lequel il est possible d'obtenir des acrylates ou méthacrylates d'alcools supérieurs de pureté élevée et à faible teneur en inhibiteurs de polymérisation et en catalyseurs, au moyen d'une transestérification des esters éthyliques ou méthyliques correspondants avec des alcools qui contiennent plus de deux atomes de carbone.

De manière surprenante, les inventeurs ont découvert que l'on peut préparer des acrylates ou méthacrylates d'alcoyle, dont les chaînes alcoyliques contiennent plus de deux atomes de carbone, de pureté élevée et à faibles concentrations d'inhibiteurs de polymérisation, lorsque la réaction de transestérification des acrylates ou méthacrylates d'éthyle ou de méthyle et des alcools supérieurs est réalisée en présence d'alcoxydes de titane comme catalyseurs et d'aminophénols ou d'aminométhylphénols comme inhibiteurs de polymérisation. De préférence, la réaction est réalisée à une température comprise entre 115 et 185 °C.

Bien que selon l'invention on puisse utiliser n'importe quel type d'amino(ou d'aminométhyl)phénols, on préfère des aminophénols ou des aminométhlphénols qui possèdent les formules structurelles suivantes:

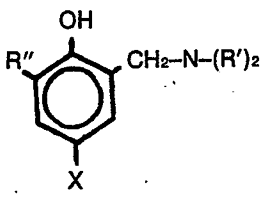
(I)

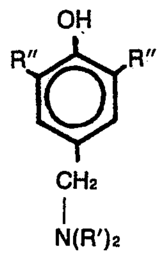
(II)

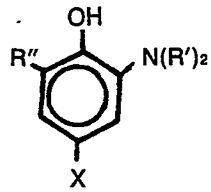
(III)

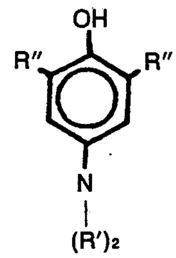
(IV)

dans lesquelles:

X = H, $CH_3$, tert-$C_4H_9$, tert-$C_5H_{11}$, $OCH_3$, Cl, Br
R' = H, $CH_3$, $CH_3$–$CH_2$–, iso-$C_3H_7$, $C_6H_5$
R" = $CH_3$, tert-$C_4H_9$, tert-$C_5H_{11}$

Les amino(ou aminométhyl)phénols et en particulier ceux qui répondent aux formules I, II, III et IV, sont des inhibiteurs extraordinairement efficaces de polymérisation des monomères acryliques et méthacryliques, en concentrations habituellement inférieures à 450 parties par million (ppm). On peut utiliser un ou plusieurs aminophénols.

L'utilisation des inhibiteurs mentionnés permet de terminer la réaction de transestérification à des températures allant jusqu'à 185° C, sans polymérisation appréciable des esters insaturés, avec l'avantage mentionné précédemment d'aboutir à des vitesses élevées de réaction et, par conséquent, des temps relativement faibles de réaction, en utilisant des concentrations de catalyseur relativement faibles inférieures à 2500 ppm et de préférence inférieures à 350 ppm. De manière évidente, la concentration minimum d'un inhibiteur nécessaire dépend dans chaque cas de la température de réaction choisie qui détermine, également, la concentration du catalyseur à utiliser pour terminer dans un temps donné une conversion déterminée. Les experts, dans chaque cas, pourront déterminer sans difficulté la concentration minimum d'inhibiteur pour quelques conditions déterminées de réaction.

Un avantage particulier de l'invention est la possibilité d'utiliser comme matière première des alcools ou des glycols de qualité technique. La teneur en eau de ces produits n'affecte pas de manière défavorable l'activité catalytique des alcoxydes de titane, étant donné qu'aux températures auxquelles se réalise le procédé, la majeure partie de l'eau quitte le réacteur de transestérification pour passer dans le distillat, sans provoquer l'hydrolyse du catalyseur.

Comme matières premières pour le procédé faisant l'objet de la présente invention, on utilise en général les esters méthyliques ou éthyliques des acides acryliques et méthacryliques.

La réaction peut avoir lieu en pratique avec n'importe quel alcool, à l'unique condition qu'il soit au moins partiellement soluble dans l'ester acrylique ou méthacrylique mis en jeu, dans les conditions de la réaction. Parmi les alcools on peut inclure les alcools aliphatiques primaires, linéaires ou ramifiés en $C_3$–$C_{20}$ tels que n-propanol, n-butanol, isobutanol, 2-éthylhexanol, alcool laurique, alcool stéarique, etc..., les glycols aliphatiques, en particulier éthylèneglycol, diéthylèneglycol, triéthylèneglycol, néopentylglycol, triméthylolpropane; les alcools aromatiques, tels que l'alcool benzylique et des alcools avec d'autres groupes fonctionnels, tels que des éther-alcools, par exemple, l'éthylèneglycolmonométhyléther ou des aminoalcools, par exemple le diéthylaminoéthanol.

On réalise la transestérification en mettant en contact l'ester et l'alcool à une température comprise entre 115 et 185 °C, dans un rapport d'acrylate ou méthacrylate, moles: équivalents –OH d'alcool, compris entre 1:1 et approximativement 6:1, en présence d'un alcoxyde de titane, et de préférence entre 2:1 et 1:1.

Bien qu'en général on peut utiliser n'importe quel type d'alcoxyde de titane, on préfère l'éthoxyde, le propoxyde et le butoxyde de titane.

Le procédé faisant l'objet de la présente invention peut être mis en œuvre dans un réacteur de transestérification doté d'une colonne de distillation, pour faciliter la séparation des alcools de faible poids moléculaire formés. Initialement, on chauffe le mélange réactionnel au reflux total, jusqu'à atteindre la température de réaction désirée et arriver en tête de colonne de distillation à la température d'ébullition de l'azéotrope d'acrylate ou méthacrylate de méthyle ou d'éthyle et de l'alcool méthylique ou éthylique, respectivement. On continue la réaction jusqu'à ce qu'on termine la distillation de l'azéotrope, ce qui indique que la réaction de transestérification est terminée. On peut éliminer l'excès d'acrylate ou méthcrylate de méthyle ou d'éthyle, selon les

techniques connues, par distillation à entraînement avec de la vapeur d'eau ou au moyen d'une distillation sous vide. Dans le cas de l'emploi de l'entraînement à la vapeur, on peut produire simultanément l'hydrolyse de l'alcoxyde de titane et, dans ce cas, on peut éliminer l'hydroxyde de titane formé par filtration ou centrifugation.

Pendant la réalisation de la réaction, on peut introduire éventuellement dans le réacteur une partie des substances réactives, par exemple les acrylates ou méthacrylates de méthyle ou d'éthyle et les alcools, dans le cas où on ne les a pas chargés dans leur totalité initialement.

Etant donné que le procédé de la présente invention est réalisé à des températures relativement élevées comprises entre 115 et 185° C, les concentrations de catalyseur nécessaire pour atteindre des vitesses élevées de réaction et, par concéquent, les temps de réaction économiquement acceptables, sont généralement très faibles, normalement inférieures à 2500 ppm. De ce fait, habituellement il n'est pas nécessaire d'éliminer le catalyseur des acrylates ou méthacrylates supérieurs obtenus, avec l'avantage qui en résulte, puisque sa présence dans les concentrations indiquées n'est pas défavorable pour la majorité des applications des acrylates et méthacrylates d'alcoyle supérieurs. Ainsi, par exemple, quand on les utilise pour la fabrication d'additifs d'huiles lubrifiantes.

Egalement, étant donné que les quantités d'aminophénols utilisés comme inhibiteurs de polymérisation sont inférieures à 450 ppm, il n'est pas nécessaire de réaliser leur élimination des acrylates ou méthacrylates d'alcoyle supérieurs obtenus selon le procédé de l'invention, de sorte qu'ils peuvent être utilisés, sans purification postérieure, pour l'obtention de différents dérivés d'intérêt industriel.

Bien qu'elle ne soit pas nécessaire dans le procécé revendiqué selon l'invention, l'élimination du méthanol ou de l'éthanol produit dans la réaction de transestérification peut faciliter l'introduction dans le réacteur d'un agent qui soit capable de former des azéotropes avec les alcools mentionnés, par exemple, le cyclohexane, conformément aux techniques bien connues.

Bien que selon le procédé constituant l'invention, il ne soit pas nécessaire d'éliminer les alcoxydes de titane utilisés comme catalyseurs, étant donné qu'ils sont utilisés en quantités très réduites, dans le cas où il serait souhaitable de faire une élimination totale, au moment de la fin de la réaction de transestérification on ajoute de l'eau à la masse en réaction et on maintient une température de 70–95° C pendant quelque 60 minutes, pour hydrolyser l'alcoxyde de titane et postérieurement on filtre, en procédant ensuite à une séparation de l'excès d'acrylate ou de méthacrylate de méthyle ou d'éthyle au moyen d'une distillation sous vide ou d'un entraînement à la vapeur d'eau, comme cela est décrit dans le brevet allemand 2.317.226, 1974.

On peut réaliser le procédé objet de la présente invention en continu ou en discontinu, comme cela est connu dans la technique actuelle.

L'invention sera maintenant décrite en référence à quelques exemples donnés simplement à titre d'illustration et qui ne sauraient donc en aucune façon limiter la portée de l'invention.

Exemple 1

Pour l'obtention d'un mélange de méthacrylates de dodécyle et tétradécyle, on introduit dans un matras en fonte rond d'un litre de capacité, doté d'une colonne de distillation de 10 plateaux théoriques et d'un système d'agitation, 386 grammes d'un mélange d'alcools dodécylique et tétradécylique de composition:

| | |
|---|---|
| alcool n-décylique | 1,0% |
| alcool n-dodécylique | 53,5% |
| alcool n-tétradécylique | 44,2% |
| alcool n-hexadécylique | 1,3% |

Tout de suite après on ajoute 107,3 grammes de méthacrylate de méthyle et 0,18 grammes du composé I ($R' = CH_3$; R″ et $X = tert\text{-}C_4H_9$).

Sous agitation, on chauffe le mélange à 100 °C. A cette température on ajoute 0,39 g de butoxyde de titane comme catalyseur et on poursuit le chauffage jusqu'à 125° C. Lorsqu'on atteint cette température on ajoute pendant 2 heures 292,6 g de méthacrylate de méthyle. A la tête de la colonne de distillation on sépare un mélange azéotrope à point d'ébullition de 65° C. Après 3 heures depuis le début de la réaction l'azéotrope cesse de distiller, en indiquant la fin de la réaction. Tout de suite, pendant 20 minutes, on réduit le vide à 40 cm de Hg, en maintenant la température de 125° C dans la masse réactionnelle pour séparer au moyen de la distillation l'excès de méthacrylate de méthyle n'ayant pas réagi. On a obtenu ainsi 510 g de méthacrylates de dodécyle-tétradécyle, ce qui suppose un rendement de 98,3% par rapport aux alcools utilisés, qui contient 350 ppm du composé (I) et 750 ppm de butoxyde de titane.

Exemple 2

Pour l'obtention d'un mélange de méthacrylates d'hexadécyle et d'octadécyle on a chargé dans un matras de 2 litres de capacité, pourvu d'une colonne de 5 plateaux théoriques et d'un système d'agitation, 700 g d'un mélange d'alcools hexadécylique et octadécylique de composition:

| | |
|---|---|
| alcool tétradécylique | 1,0% |
| alcool n-hexadécylique | 48,0% |
| alcool n-octadécylique | 49,2% |
| alcool n-$C_{20}H_{41}$–OH | 1,8% |

Sous agitation, on chauffe ce mélange d'alcools à 145° C, on ajoute 0,22 g de II ($R'' = tert\text{-}C_5H_{11}$, $R' = CH_3$) et 0,22 g de butoxyde de titane dissous dans 50 g de méthacrylate de méthyle. Ensuite et pendant 2 heures on ajoute 387,3 g de méthacrylate de méthyle, en maintenant une température constante dans le réacteur à 145° C. En tête de colonne de distillation on sépare un mélange azéotrope de point d'ébullition 65° C (75,3% de méthanol, 24,7% de méthacrylate de méthyle). Au bout de 2,5 heures à partir de l'addition du butoxyde de titane, le mélange azéotrope cesse de distiller, ce qui indique la fin de la réaction. Ensuite, on introduit dans le réacteur un

petit courant de vapeur, en séparant en tête de colonne de distillation l'excès de méthacrylate de méthyle n'ayant pas réagi. Ce distillat se condense et se refroidit pour former deux nappes d'eau et de méthacrylate de méthyle; ce dernier peut être recyclé au réacteur. On a obtenu ainsi 878 g de méthacrylates d'hexadécyle-octadécyle, avec un rendement de 99% par rapport aux alcools initiaux, qui contiennent 250 ppm du composé (II) et du butoxyde de titane, soit 0,012% d'acide méthacrylique et 0,09% d'eau.

Exemple 3

On dispose d'un matras à fond rond de 500 cm³ de capacité, doté d'un thermomètre, d'un système d'agitation et d'une colonne de fractionnement «vigreus», dont à la partie supérieure on accouple un réfrigérant pour la condensation des vapeurs. On charge dans le réacteur 71,7 g de 2-éthylhexanol et 0,031 g du composé II (R″ = tert-$C_4H_9$, R′ = $CH_3$). Ensuite on élève la température jusqu'à 125 °C et on introduit 0,037 g d'isopropoxyde de titane et 83,8 g d'acrylate de méthyle pendant 3 heures, en maintenant la température constante pendant ce temps, au moyen d'un chauffage externe. A la tête de la colonne, on distille un mélange azéotrope méthanol/acrylate de méthyle de point d'ébullition 62–63° C. On maintient le mélange réactionnel une heure additionnelle à 125° C, finalement on distille sous vide l'excès d'acrylate de méthyle n'ayant pas réagi. On a obtenu 99,7 g d'acrylate de 2-éthylhexyle avec une teneur de 310 ppm du composé (II), ce qui suppose un rendement de 98,5%.

**Revendications**

1. Procédé d'obtention d'acrylates ou de méthacrylates d'alcoyle supérieurs, dont les chaînes alcoyliques ont plus de deux atomes de carbone, qui contiennent de faibles concentrations d'inhibiteurs de polymérisation et de catalyseurs, par transestérification d'acrylates ou de méthacrylates de méthyle ou d'éthyle avec les alcools supérieurs correspondants, en présence d'alcoxydes de titane comme catalyseurs et d'inhibiteurs de polymérisation et, éventuellement, d'un agent pour faciliter la séparation azéotrope des alcools méthyliques ou éthyliques produits dans la réaction, caractérisé en ce qu'on utilise comme inhibiteurs de polymérisation des aminophénols ou des aminométhylphénols.

2. Procédé selon la revendication 1, caractérisé en ce qu'on utilise au moins un des amino(ou aminométhyl)phénols correspondant aux formules structurelles suivantes:

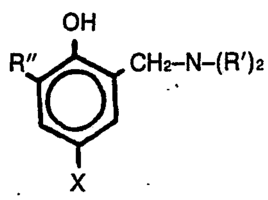

(I)

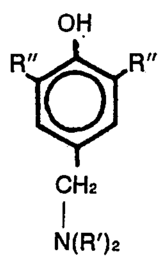

(II)

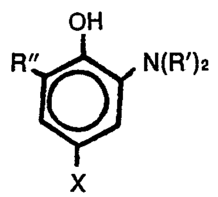

(III)

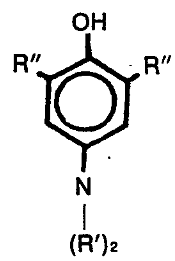

(IV)

dans lesquelles:

X = H, $CH_3$, tert-$C_4H_9$, tert-$C_5H_{11}$, $OCH_3$, Cl, Br
R′ = H, $CH_3$, $CH_3$–$CH_2$–, iso-$C_3H_7$, $C_6H_5$
R″ = $CH_3$, tert-$C_4H_9$, tert-$C_5H_{11}$

3. Procédé selon la revendication 1 ou 2, caractérisé en ce qu'on réalise la réaction de transestérification à des températures comprises entre approximativement 115–185° C et, de préférence, entre 125–150° C.

4. Procédé selon l'une des revendications 1 à 3, caractérisé en ce qu'on réalise la transestérification avec un rapport molaire acrylate ou méthacrylate de méthyle ou d'éthyle par rapport aux groupes alcooliques, qui est compris entre 6:1 et 1:1 et de préférence entre 2:1 et 1:1.

5. Procédé selon l'une des revendications 1 à 4, caractérisé en ce que l'on utilise l'alcoxyde de titane employé comme catalyseur en des concentrations inférieures à 2500 parties par million et, de préférence, en des concentrations inférieures à 350 parties par million.

6. Procédé selon l'une des revendications 1 à 5, caractérisé en ce que l'on emploie les amino(ou aminométhyl)phénols utilisés comme inhibiteurs de polymérisation en des concentrations inférieures à 450 parties par million.

**Patentansprüche**

1. Verfahren zur Gewinnung von höheren Alkyl-Acrylaten oder Methacrylaten mit Alkylketten von mehr als zwei Kohlenstoffatomen, die geringe Konzentrationen von Polymerisationsinhibitoren enthalten, durch Transveresterung von Methyl- oder Ethylacrylaten oder Methacrylaten mit entsprechenden höheren Alkoholen, in Anwesenheit von Titanalcoxyden als Katalysatoren und von Polymerisationsinhibitoren und gegebenenfalls von einem Mittel, das die azeotrope Abtrennung der durch die Reaktion gewonnenen Methyl- oder Ethylalkohole erleichtert, dadurch gekennzeichnet, dass Aminophenole oder Aminomethylphenole als Polymerisationsinhibitoren verwendet werden.

2. Verfahren nach Anspruch 1, dadurch gekennzeichnet, dass mindestens eines von den Aminophenolen oder Aminomethylphenolen entsprechend der folgenden strukturellen Formel:

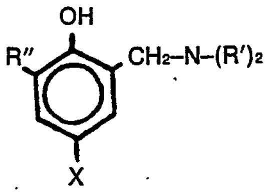
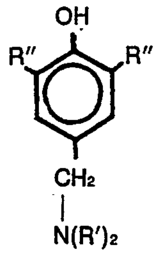
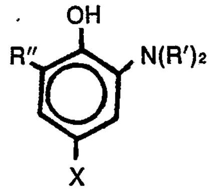
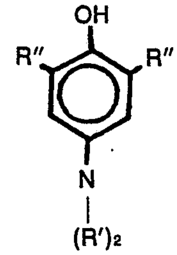

(I) (II) (III) (IV)

worin:

X = H, $CH_3$, tert-$C_4H_9$, tert-$C_5H_{11}$, $OCH_3$ Cl, Br
R′ = H, $CH_3$, $CH_3$–$CH_2$–, iso-$C_3H_7$, $C_6H_5$
R″ = $CH_3$, tert-$C_4H_9$, tert-$C_5H_{11}$

verwendet wird.

3. Verfahren nach Anspruch 1 oder 2, dadurch gekennzeichnet, dass die Transveresterungsreaktion bei einer Temperatur zwischen ungefähr 115–185° C und, vorzugsweise zwischen 125–150° C, durchgeführt wird.

4. Verfahren nach einem der Ansprüche 1 bis 3, dadurch gekennzeichnet, dass die Transveresterung mit einem molarischen Verhältnis von Methyl- oder Ethylacrylat oder -Methacrylat gegenüber den Alkoholgruppen zwischen 6:1 und 1:1 und vorzugsweise zwischen 2:1 und 1:1, durchgeführt wird.

5. Verfahren nach einem der Ansprüche 1 bis 4, dadurch gekennzeichnet, dass Titanalkoxyd als Katalysator in Konzentrationen unter 2500 Teile pro Million und, vorzugsweise, in Konzentrationen unter 350 Teile pro Million (ppm), verwendet wird.

6. Verfahren nach einem der Ansprüche 1 bis 5, dadurch gekennzeichnet, dass Aminophenole oder Aminomethylphenole als Polymerisationsinhibitoren in Konzentrationen unter 450 Teile pro Million verwendet werden.

**Claims**

1. Method for obtaining higher alkyl acrylates or methacrylates, whose alkyl chains have more than two carbon atoms, which contain low concentrations of polymerization inhibitors and of catalysts, through the transesterification of methyl or ethyl acrylates or methacrylates with the corresponding higher alcohols, in the presence of titanium alcoxides as catalysts and of polymerization inhibitors and, possibly, of an agent for facilitating the azeotropic removal of the methyl or ethyl alcohols produced during the reaction, characterized in that aminophenols or aminomethylphenols are used as polymerization inhibitors.

2. Method according to claim 1, characterized in that use is made of at least one of the aminophenols or aminomethylphenols corresponding to the following structural formulae:

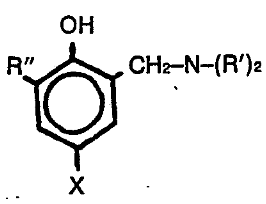
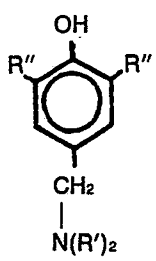
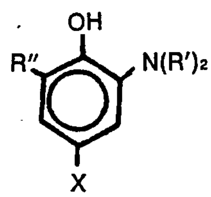
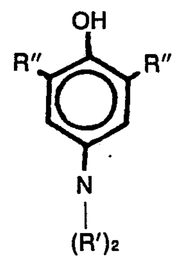

(I) (II) (III) (IV)

wherein:

X = H, $CH^3$, tert-$C_4H_9$, tert-$C_5H_{11}$, $OCH_3$, Cl, Br
R′ = H, $CH_3$, $CH_3$–$CH_2$–, iso-$C_3H_7$, $C_6H_5$
R″ = $CH_3$, tert-$C_4H_9$, tert-$C_5H_{11}$

3. Method according to claim 1 or 2, characterized in that the reaction of transesterification is performed at temperatures comprised between approximately 115 and 185° C, and preferably between 125 and 150° C.

4. Method according to one of claims 1 to 3, characterized in that the transesterification is performed with a methyl or ethyl acrylate or methacrylate molar ratio with respect to the alcoholic groups, which is comprised between 6:1 and 1:1 and preferably between 2:1 and 1:1.

5. Method according to one of claims 1 to 4, characterized in that the titanium alcoxide used as catalyst is used in concentrations lower than 2500 parts per million and, preferably, in concentrations lower than 350 parts per million.

6. Method according to one of claims 1 to 5, characterized in that the aminophenols or aminomethylphenols used as polymerization inhibitors are used in concentrations lower than 450 parts per million.